# EUROPÄISCHE PATENTANMELDUNG

(11) **EP 4 847 015 A1**
(43) Veröffentlichungstag der Anmeldung: **02.12.2026**
(21) Anmeldenummer: 25225419.8
(22) Anmeldetag: 19.12.2025
(51) Int. Cl.: A61K 31/135, A61K 31/192, A61K 31/495, A61K 31/522, A61P 25/32

(54) **ZUSAMMENSETZUNG ENTHALTEND NSAR, DIMENHYDRINAT UND CINNARIZIN ZUR VERWENDUNG BEI DER BEHANDLUNG VON VEISALGIA**

(71) Anmelder: Senska, Götz, 45133 Essen (DE)
(72) Erfinder: Senska, Götz, 45133 Essen (DE)
(74) Vertreter: Von Rohr Patentanwälte Partnerschaft mbB

(57) **Zusammenfassung**

Die vorliegende Erfindung betrifft das Gebiet der Medizin und insbesondere das technische (d. h. medizinisch-pharmazeutische) Gebiet der Behandlung bzw. Therapie von Veisalgia (Alkoholintoxikation, *Hangover*). Insbesondere betrifft die vorliegende Erfindung eine Zusammensetzung, insbesondere pharmazeutische Zusammensetzung, welche zur Verwendung bei der prophylaktischen bzw. therapeutischen (symptomatischen) Behandlung von Veisalgia (Alkoholintoxikation, *Hangover*) geeignet bzw. vorgesehen ist.

## Beschreibung

Die vorliegende Erfindung betrifft das Gebiet der Medizin und insbesondere das technische (d. h. medizinisch-pharmazeutische) Gebiet der Behandlung bzw. Therapie von Veisalgia (Alkoholintoxikation, *Hangover).*

Insbesondere betrifft die vorliegende Erfindung eine Zusammensetzung, insbesondere pharmazeutische Zusammensetzung, welche zur Verwendung bei der prophylaktischen bzw. therapeutischen (symptomatischen) Behandlung von Veisalgia (Alkoholintoxikation, *Hangover)* geeignet bzw. vorgesehen ist.

Die Indikation Veisalgia, d. h. der Zustand der Alkoholintoxikation, welcher auch als *"Hangover"* oder "Kater" bezeichnet wird, beschreibt den Zustand bzw. ein recht breites Symptombild nach übermäßigem Alkoholkonsum, der bzw. das typischerweise mehrere Stunden nach Abklingen der akuten Rauschwirkung einsetzt. Übliche Symptome sind u. a. Kopfschmerzen, Übelkeit, Müdigkeit, Schwindel, gesteigerte Empfindlichkeit sowie eine allgemeine körperliche und psychische Beeinträchtigung.

Die zugrunde liegende Pathophysiologie ist multifaktoriell und noch nicht vollständig verstanden. Es können mehrere Entstehungsmechanismen für die genannten Symptome identifiziert werden, wobei der Alkoholmetabolismus ein zentraler Punkt ist. Ferner wirkt Alkohol diuretisch und auch eine durch Alkohol verursachte Hypoglykämie, insbesondere bei leerem Magen, kann zur vorbeschriebenen Symptomatik beitragen. Eine zusätzliche Steigerung des Hangover-Potentials ergibt sich durch Kongenere, die insbesondere in dunklen Spirituosen wie Whiskey oder Rum enthalten sind. Nicht zuletzt beeinträchtigt Alkohol auch die Schlafarchitektur.

Eine kausale Therapie für *Hangover* steht bislang nicht zur Verfügung; die Behandlung erfolgt symptomatisch. Im Vordergrund stehen Maßnahmen wie eine erhöhte Flüssigkeitszufuhr, die Aufnahme kohlenhydratreicher Nahrung zur Stabilisierung des Blutzuckerspiegels und körperliche Regeneration.

Ergänzend oder auch präventiv kann die Einnahme von Arzneimitteln erfolgen, wobei hier bspw. aufgrund schädlicher Organwirkungen Einschränkungen bestehen bzw. sich reelle Gesundheitsrisiken über den Intoxikationszustand hinaus ergeben können.

So ist etwa aus dem Patentdokument EP 0 426 479 B1 eine Zusammensetzung bekannt, die zur Behandlung der Symptome von übermäßigem Genuss, insbesondere nach übermäßigem Konsum alkoholischer Getränke und/oder Nahrungsmitteln, vorgeschlagen wird. Die dort beschriebene Formulierung kombiniert bevorzugt ein Analgetikum oder Antipyretikum, bspw. Paracetamol, mit einem gastrisch aciditätshemmenden Wirkstoff, beispielsweise Cimetidin, Ranitidin, Famotidin oder Omeprazol.

Diese Kombination ist aus pharmakologisch-toxikologischer Sicht speziell für die Behandlung von *Hangovern* als problematisch anzusehen. So unterliegt Paracetamol etwa einer hepatischen Biotransformation, die insbesondere nach Alkoholkonsum über den Cytochrom-P450-abhängigen Metabolismus (CYP2E1) zu reaktiven Zwischenprodukten führt. Bei reduzierten Glutathionreserven, wie sie typischerweise nach Alkoholgenuss bestehen, kann die Entgiftung dieser Zwischenprodukte unzureichend sein, sodass es zu lebertoxischen Effekten kommt.

Darüber hinaus kann der gleichzeitig eingesetzte aciditätshemmende Wirkstoff die Magenentleerung und damit die Resorption und Metabolisierung von Nahrungs- und Alkoholrückständen verzögern. Die vorgeschlagenen H₂-Blocker und Protonenpumpen-Inhibitoren führen zu einer Erhöhung des intragastrischen pH-Wertes sowie zu einer Abnahme der proteolytischen Aktivität, wodurch der natürliche Abbau von Nahrungsbestandteilen verlangsamt wird. Dies kann die Verweildauer von Restalkohol oder Fuselalkoholmetaboliten im Magen verlängern und somit die Symptomatik des *Hangover* prolongieren oder verstärken.

Ferner kann die geänderte pH-Situation im Magen die Resorptionskinetik des Analgetikums bzw. Antipyretikums beeinflussen und zu ungleichmäßigen Plasmaspiegeln führen, wodurch die gewünschte analgetische Wirkung verspätet oder abgeschwächt eintritt.

Die vorgeschlagene Wirkstoffkombination ist daher speziell für die Indikation der Behandlung von Veisalgia letztlich nicht zweckmäßig bzw. potenziell kontraindiziert, insbesondere im Hinblick auf die Sicherheit und den physiologischen Regenerationsprozess nach Alkoholexzess. Für die Folgen übermäßigen Alkoholkonsums, insbesondere die Behandlung von Veisalgia bzw. *Hangovern,* stehen bislang also keine geeigneten systematischen bzw. auf die Indikation speziell zugeschnitten (symptomatischen) Therapien zur Verfügung.

Vor diesem Hintergrund besteht eine Aufgabe der vorliegenden Erfindung darin, ein Konzept bereitzustellen, dass die bisherigen Nachteile bzw. Mangel bestehender Ansätze zur Behandlung bzw. Minderung von Veisalgia bzw. *Hangovern* ausräumt oder aber zumindest abschwächt.

Der vorliegenden Erfindung liegt dabei insbesondere die Aufgabe zugrunde, ein verlässliches Behandlungskonzept bereitzustellen, dass die Symptomatik von Veisalgia bzw. *Hangovern* gezielt sowie kombiniert adressiert, insbesondere bei verminderten Risiko für Nebenwirkungen.

Zur Lösung der zuvor geschilderten Aufgabe schlägt die vorliegende Erfindung daher - gemäß einem **ersten** Aspekt der vorliegenden Erfindung - eine insbesondere pharmazeutische Zusammensetzung zur Verwendung bei der prophylaktischen und/oder therapeutischen (symptomatischen) Behandlung von Veisalgia (Alkoholintoxikation, *Hangover)* gemäß Anspruch 1 vor. Jeweils vorteilhafte Weiterbildungen und Ausgestaltungen dieses Erfindungsaspekts sind Gegenstand der betreffenden Unteransprüche.

Weitere Gegenstände der vorliegenden Erfindung - gemäß den **weiteren** Aspekten der vorliegenden Erfindung - sind die Verwendung einer insbesondere pharmazeutischen Zusammensetzung nach der vorliegenden Erfindung zur Herstellung eines Arzneimittels oder Medikaments zur prophylaktischen und/oder therapeutischen (symptomatischen) Behandlung von Veisalgia sowie ein entsprechendes Arzneimittel oder Medikament gemäß dem diesbezüglichen unabhängigen Anspruch.

Es versteht sich von selbst, dass Ausgestaltungen, Ausführungsformen, Vorteile und dergleichen, welche nachfolgend zur Vermeidung von Wiederholungen nur zu einem Erfindungsaspekt aufgeführt sind, selbstverständlich auch in Bezug auf die übrigen Erfindungsaspekte gelten, ohne dass dies einer gesonderten Erwähnung bedarf.

Weiterhin versteht es sich von selbst, dass bei nachfolgenden Angaben von Werten, Zahlen und Bereichen die diesbezüglichen Werte-, Zahlen- und Bereichsangaben nicht beschränkend zu verstehen sind; es versteht sich für den Fachmann von selbst, dass einzelfallbedingt oder anwendungsbezogen von dem angegebenen Bereich bzw. Angaben abgewichen werden kann, ohne dass der Rahmen der vorliegenden Erfindung verlassen ist.

Ferner ist bei allen nachstehend genannten relativen bzw. prozentualen, insbesondere gewichtsbezogenen Mengenangaben zu beachten, dass diese Angaben im Hinblick auf das herangezogene Bezugssystem (z. B. Darreichungsform bzw. Zusammensetzung) vom Fachmann derart auszuwählen bzw. zu kombinieren sind, dass in der Summe - gegebenenfalls unter Einbeziehung weiterer Komponenten bzw. Inhaltsstoffe bzw. Zusatzstoffe bzw. Bestandteile, insbesondere wie nachfolgend definiert - stets 100 % bzw. 100 Gew.-% resultieren. Dies versteht sich für den Fachmann aber von selbst.

Zudem gilt, dass alle im Folgenden genannten Werte- bzw. Parameterangaben oder dergleichen grundsätzlich mit genormten bzw. standardisierten oder explizit angegebenen Bestimmungsverfahren oder aber mit dem Fachmann auf diesem Gebiet an sich geläufigen Bestimmungsmethoden ermittelt bzw. bestimmt werden können.

Darüber hinaus gilt für die Beschreibung der vorliegenden Erfindung, dass die jeweils im Zusammenhang mit den speziellen Ausgestaltungen, Ausführungsformen, Vorteilen, Beispielen oder dergleichen angeführten Merkmale der vorliegenden Erfindung auch in deren Kombination als offenbart gelten. Somit gelten vorliegend auch übergeordnete Kombinationen einzelner oder mehrerer Merkmale, welche für jeweilige Ausgestaltungen, Ausführungsformen, Anwendungsbeispiele oder dergleichen angeführt sind, als offenbart.

Insbesondere gilt für die die Erfindung charakterisierenden Merkmale, dass sämtliche Kombinationsmöglichkeiten dieser Merkmale als offenbart gelten, wobei Ausführungsformen vergleichbarer bzw. entsprechender Präferenz der verschiedenen Merkmale in ihrer Kombination bevorzugt sind (z.B. Mengen bzw. Mengenbereiche der betreffenden Wirk- und Inhaltsstoffe gleicher Präferenz oder dergleichen).

Dabei gilt insbesondere auch, dass für die nachfolgend angeführten, die verschiedenen Wirk- bzw. Inhaltsstoffe der erfindungsgemäßen Zusammensetzung betreffenden Mengenangaben, insbesondere relativen Mengenangaben oder absoluten Mengenangaben gleicher Präferenz bzw. gleicher Bevorzugungsebene, jeweilige und die verschiedenen Wirk- bzw. Inhaltsstoffe betreffende Kombinationen mit entsprechender Präferenz bzw. Bevorzugung mitoffenbart sind. Ebenfalls sind auch sämtliche anderweitige Kombinationen (d.h. Kombinationen auf Basis unterschiedlicher Präferenzen bzw. unterschiedlicher Bevorzugungsebenen) mitoffenbart.

Dies vorausgeschickt, wird die vorliegende Erfindung nunmehr nachfolgend im Detail erläutert:
Gegenstand der vorliegenden Erfindung - gemäß einem **ersten** Aspekt der vorliegenden Erfindung - ist ein Zusammensetzung, insbesondere pharmazeutische Zusammensetzung, zur Verwendung bei der prophylaktischen und/oder therapeutischen (symptomatischen) Behandlung von Veisalgia (Alkoholintoxikation, *Hangover),*
wobei die Zusammensetzung jeweils eine wirksame, insbesondere pharmazeutisch und/oder therapeutisch wirksame, Menge von Wirkstoffen aus den Wirkstoffklassen (i) nichtsteroidales Antirheumatikum (NSAR, *non-steroidal anti-inflammatory drug,* NSAID), (ii) H₁-Antihistaminikum und (iii) Calciumkanal-Antagonist umfasst, wobei die Wirkstoffe ausgewählt sind aus
Klasse (i): Acetylsalicylsäure, Ibuprofen, Dexibuprofen, Flurbiprofen, Naproxen,
   Ketoprofen, Tiaprofensäure, Diclofenac, Indometacin, Acemetacin, Flufenaminsäure, Mefenaminsäure, Piroxicam, Tenoxicam, Meloxicam, Lornoxicam, Nabumeton und/oder Mischungen hiervon,
Klasse (ii): Dimenhydrinat, und
Klasse (iii): Cinnarizin.

Im Rahmen dieser Anmeldung soll die Angabe eines Wirkstoffs, insbesondere wie vorstehend, unter dessen Freibezeichnung bzw. Freiname sämtliche pharmazeutisch verträglichen Formen dieses Wirkstoffs miteinschließen, insbesondere Salze, Ester, Amide, Solvate, Hydrate, polymorphe Modifikationen, Isomere, Enantiomere und Prodrugs, soweit diese die gleiche oder eine funktionell äquivalente pharmakologische Aktivität aufweisen.

Die Begriffe "Salz" bzw. "Ester", wie sie vorliegend im Hinblick auf die Wirk- bzw. Inhaltsstoffe angeführt werden, beziehen sich insbesondere auf die jeweiligen pharmazeutisch kompatiblen bzw. physiologisch verträglichen bzw. unbedenklichen Salze bzw. Ester der angeführten Wirk- bzw. Inhaltsstoffe.

Der Begriff "Zusammensetzung" bzw. "pharmazeutische Zusammensetzung", wie dieser im Rahmen der vorliegenden Erfindung verwendet wird, ist darüber hinaus breit zu verstehen und umfasst nicht nur pharmazeutische Präparate bzw. Pharmazeutika und Arzneimittel als solche, sondern soll auch sogenannte Medizinprodukte, homöopathische Mittel und Nahrungsergänzungsmittel etc. miterfassen.

Im Hinblick auf die vorliegende Erfindung hat die Anmelderin in völlig überraschender Weise gefunden, dass mit der erfindungswesentlichen Kombination aus Wirkstoffen der Wirkstoffklassen (i) nichtsteroidales Antirheumatikum (NSAR, *non-steroidal anti-inflammatory drug,* NSAID), (ii) H₁-Antihistaminikum und (iii) Calciumkanal-Antagonist insbesondere pharmazeutische Zusammensetzungen mit einem für die Behandlung von Veisalgia bzw. *Hangovern* optimierten Wirkprofil erhalten werden.

Die Wirkstoffe der Klasse (i) sind Verbindungen mit analgetischer, antipyretischer und antiinflammatorischer Wirkung, die ihre pharmakologische Aktivität im Wesentlichen durch Hemmung der Cyclooxygenase-Enzyme (COX-1 und/oder COX-2) entfalten und dadurch die Biosynthese von Prostaglandinen, Prostacyclinen und Thromboxanen hemmen. Sie stellen für die angedachte Indikation eine pharmakologisch sichere, schnell wirksame und physiologisch kompatible Wirkstoffklasse dar. Insbesondere zeichnen sich die Wirkstoffe durch eine breite Wirksamkeit gegenüber der zu behandelnden Symptomatik, eine vergleichsweise geringe hepatische Belastung und eine optimierte Integrationsfähigkeit in insbesondere auch magenschonende Formulierungen aus.

Diese Wirkstoffe können einzeln oder in Kombination miteinander verwendet werden. Die Auswahl des jeweiligen Wirkstoffs erfolgt generell in Abhängigkeit von der gewünschten Wirkstärke, der Pharmakokinetik, der Verträglichkeit und der beabsichtigten Indikation. Erfindungsgemäß bevorzugte Vertreter sind Ibuprofen, Dexibuprofen und/oder Naproxen aufgrund ihres günstigen Verträglichkeitsprofils und ihrer breiten therapeutischen Anwendung.

Weiter bevorzugt ist Diclofenac, welches über eine ausgeprägte antiinflammatorische Wirksamkeit verfügt. Acetylsalicylsäure ist insbesondere aufgrund ihrer analgetischen und antipyretischen Effekte vorteilhaft einsetzbar.

Das erfindungsgemäß eingesetzte H₁-Antihistaminikum (ii) Dimenhydrinat stellt ein Salz aus Diphenhydramin und 8-Chlortheophyllin dar und kombiniert in diesem Sinne zwei Wirkstoffe. Diphenhydramin blockiert dabei Histamin-H₁-Rezeptoren und wirkt damit auf das zentrale Nervensystem ein. Auf diese Weise kann es gegen Übelkeit und Erbrechen sowie als Beruhigungsmittel wirken. 8-Chlortheophyllin aus der chemischen Klasse der Xanthine wirkt als Stimulans mit koffeinähnlichen physiologischen Wirkungen. Auf diese Weise mindert es den sedativen Effekt von Diphenhydramin und trägt auch darüber hinaus durch die anregende Wirkung zur Milderung der Symptomatik von *Hangovern* bei.

Dimenhydrinat wird herkömmlich zur Behandlung von Reiseübelkeit eingesetzt. Reiseübelkeit entsteht, wenn im Gehirn widersprüchliche Informationen von den Sinnesorganen zusammenlaufen. Der resultierende Sinneskonflikt überfordert das Gehirn, was zur Aktivierung des Brechzentrums und zu Symptomen wie Übelkeit führen kann. In diesem Sinne weicht der herkömmliche Einsatz von Dimenhydrinat bzw. die damit intendierte Symptomlinderung maßgeblich von der erfindungsgemäß angedachten Behandlung von Veisalgia, die ihrerseits auf einem Intoxikationszustand und damit einer tatsächlichen körperlichen Beeinträchtigung beruht, ab.

Als Calciumkanal-Antagonist (iii) ist erfindungsgemäß die Verwendung von Cinnarizin vorgesehen. Herkömmlich dient es zur Behandlung von Störungen der zerebralen und peripheren Durchblutung sowie von Gleichgewichtsproblemen (Schwindel). Es ist ein Piperazin-Derivat, das die zelluläre Calciumüberladung reguliert und dadurch u.a. eine Erweiterung der Blutgefäße fördern kann.

Die im Rahmen der Erfindung vorgesehene Wirkstoffkombination adressiert vorteilhaft gleichzeitig inflammatorische, neurovegetative und vaskuläre Mechanismen. Derartige Mechanismen sind an komplexen Symptomzuständen wie sie insbesondere im Zusammenhang mit Veisalgia bzw. *Hangovern* auftreten beteiligt und typischerweise aufwendig bzw. schwer zu adressieren.

Im Rahmen der Erfindung wird aufgrund der vorgeschlagenen Wirkstoffkombination vorteilhaft eine multimodale und synergistische pharmazeutische Wirkung realisiert, ohne dass pharmakologisch redundante Überlappungen auftreten. Die synergistische Wirkung resultiert aus einer peripheren Schmerz- bzw. Entzündungshemmung, während histaminvermittelte Symptome und neurovegetative Reaktionen moduliert und vaskuläre sowie neuronale Erregungsprozesse durch eine Blockade spannungsabhängiger Ca²⁺-Kanäle stabilisiert werden.

Im Detail können die NSARs (i) über die Cyclooxygenase-Hemmung (COX-1/COX-2) die Prostaglandinsynthese reduzieren, wodurch periphere Entzündungsprozesse, Schmerzrezeptor-Sensitivität und vaskuläre Dilatation vermindert werden. Der Calciumkanal-Antagonist (iii) kann diesen Effekt ergänzen, indem eine Calciumvermittelte Neurotransmitterfreisetzung gehemmt und damit neurogene Entzündungs- und Schmerzkomponenten abschwächt werden. Diese Kombination ermöglicht eine potenzierte analgetische Wirkung bei möglicher geringerer NSAR-Dosierung, was die gastrointestinale Belastung reduziert.

Darüber hinaus kann die Kombination von H₁-Antihistaminikum (ii) und Calciumkanal-Antagonist (iii) eine gesteigerte Blockade der vestibulären Reizleitung erzielen, wodurch das System gegen Bewegungs- und Alkohol-induzierte Irritationen stabilisiert wird.

Darüber hinaus beruhen viele *Hangover-Symptome* auf vaskulären Dysregulationen (bspw. Weitstellung zerebraler Gefäße gesteigerte Permeabilität). Der Calciumkanal-Antagonist (iii) kann hier die intrazelluläre Ca²⁺-Influx-vermittelte Vasodilatation hemmen, während die NSARs (i) zur Senkung der prostaglandinabhängigen Gefäßpermeabilität beitragen. In dieser Kombination bewirken die Wirkstoffe vorteilhaft eine Normalisierung des Gefäßtonus sowie eine Reduktion vaskulär bedingter Kopfschmerzen. Das H₁-Antihistaminikum (ii) trägt hierzu durch eine Stabilisierung der Endothelbarriere und Hemmung histaminvermittelter Vasodilatation zusätzlich bei.

Indem die Wirkstoffklassen (i) bis (iii), wie vorstehend erläutert, an unterschiedlichen, komplementären Zielstrukturen wirken, wird vorteilhaft eine geringe pharmakodynamische Überlappung erreicht, was vorteilhaft auch eine gegenseitige Dosisreduktion bei gleichbleibender Wirksamkeit ermöglicht.

Zudem können auch Magenreizungen abgemildert werden sowie eine gleichmäßige periphere Durchblutung, welche die generelle Resorption und Distribution verbessert, realisiert werden.

Insgesamt gestattet die erfindungsgemäß vorgesehene Kombination von Wirkstoffklassen (i) bis (iii), insbesondere auf Basis der spezifisch vorgesehenen Wirkstoffe, vorteilhaft eine multimodale Behandlung der komplexen Symptomzustände infolge der toxischen Belastungen durch Alkoholintoxikation bzw. Veisalgia. Durch die unterschiedlichen und gleichwohl komplementären Wirkmechanismen können insgesamt vorteilhaft analgetische, antiinflammatorische, vasostabilisierende und antiemetische Effekte erzielt werden - und dies insbesondere bei insgesamt vergleichsweise hoher Verträglichkeit und potentiell reduzierter Einzelwirkstoffdosis. Es ergibt sich damit insgesamt eine unerwartete synergistische Wirkweise der erfindungsgemäßen Zusammensetzung für die intendierte Indikation, so dass mit der Erfindung vorteilhaft eine gezielte sowie verbesserte Behandlung von *Hangovern* möglich wird.

Zu den Wirk- und ggf. weiteren Inhaltsstoffen der beanspruchten Zusammensetzung sei weiterführend auch Folgendes ausgeführt:
Die erfindungsgemäße Zusammensetzung enthält die Wirkstoffe der Klassen (i) und (ii) vorzugsweise in gewichtsbezogen Mengenverhältnissen Klasse (i) : Klasse (ii) im Bereich von 1 : 2 bis 20 : 1, insbesondere 2 : 1 bis 15 : 1, vorzugsweise 5 : 1 bis 10 : 1. Gleichermaßen bevorzugt enthält die Zusammensetzung die Wirkstoffe der Klassen (i) und (iii) in gewichtsbezogen Mengenverhältnissen Klasse (i) : Klasse (iii) im Bereich von 1 : 1 bis 30 : 1, insbesondere 3 : 1 bis 25 : 1, vorzugsweise 10 : 1 bis 20 : 1. Gute Ergebnisse im Hinblick auf das realisierte Wirkspektrum der erfindungsgemäßen Zusammensetzung werden ebenfalls erhalten, wenn die Zusammensetzung die Wirkstoffe der Klassen (ii) und (iii) in gewichtsbezogen Mengenverhältnissen Klasse (ii) : Klasse (iii) im Bereich von 5 : 1 bis 1 : 1, insbesondere 3 : 1 bis 1 : 1,25, vorzugsweise 2,5 : 1 bis 1,5 : 1, enthält.

Im Rahmen einer bevorzugten Ausführung der Erfindung ist es vorgesehen, dass die Zusammensetzung die Wirkstoffe der Klassen (i), (ii) und (iii) in gewichtsbezogen Mengenverhältnissen Klasse (i) : Klasse (ii) : Klasse (iii) im Bereich von 30 : 3 : 1 bis 3 : 1 : 1, insbesondere 25 : 2,5 : 1 bis 10 : 1,25 : 1, vorzugsweise 20 : 2,5 : 1 bis 15 : 1,5 : 1, enthält.

Für diese Ausgestaltung wird eine optimale Wirkkombination der Wirkstoffe der Klassen (i) bis (iii) realisiert, so dass eine effektive und anhaltende Behandlung der Hangover-Symptomatik möglich ist.

Für erfindungsgemäße Zusammensetzung mit Mengen der Wirkstoffe der Klassen (i) bis (iii) in den vorgenannten Mengenverhältnisbereichen werden die eingangs beschriebenen vorteilhaften Effekte der erfindungsgemäßen Zusammensetzung in besonderem Maße realisiert.

Weiter hat es sich als zweckmäßig erwiesen, wenn der Wirkstoff der Klasse (i) ausgewählt ist aus Acetylsalicylsäure, Ibuprofen, Dexibuprofen, Naproxen, Diclofenac und/oder Mischungen hiervon. Bevorzugt ist der Wirkstoff der Klasse (i) Ibuprofen. Die vorgenannten NSARs bzw. NSAIDs zeichnen sich durch eine etablierte schmerz- und entzündungshemmende Wirkung aus, die insbesondere auch im Zusammenhang mit der Indikation Veisalgia zuverlässig eintritt. Gleichzeitig ist das Risiko für pharmakologisch-toxikologische Effekte minimiert bzw. tolerabel, insbesondere auch bei Einnahme höherer Dosen der vorgerannten Wirkstoffe.

Die erfindungsgemäße Zusammensetzung enthält die Wirkstoffe insgesamt vorzugsweise in Mengen in einem Bereich von 40 Gew.-% bis 98 Gew.-%, insbesondere 45 Gew.-% bis 96 Gew.-%, vorzugsweise 50 Gew.-% bis 95 Gew.-%, bezogen auf die Gesamtzusammensetzung.

Für eine effektive Wirkung und Behandlung der Hangover-Symptomatik hat es sich weiter bewährt, wenn die Zusammensetzung Wirkstoffe der Klasse (i) in Mengen in einem Bereich von 30 Gew.-% bis 75 Gew.-%, insbesondere 40 Gew.-% bis 70 Gew.-%, vorzugsweise 50 Gew.-% bis 65 Gew.-%, bezogen auf die Gesamtzusammensetzung, enthält.

Wirkstoffe der Klasse (ii) sind in der erfindungsgemäßen Zusammensetzung vorzugsweise in Mengen in einem Bereich von 4 Gew.-% bis 20 Gew.-%, insbesondere 5 Gew.-% bis 15 Gew.-%, vorzugsweise 6 Gew.-% bis 10 Gew.-%, bezogen auf die Gesamtzusammensetzung, enthalten.

Für Wirkstoffe der Klasse (iii) hat es sich als vorteilhaft im Hinblick auf die Behandlung der angestrebten Symptomatik erwiesen, wenn diese in der Zusammensetzung in Mengen in einem Bereich von 0,5 Gew.-% bis 7 Gew.-%, insbesondere 1,5 Gew.-% bis 5 Gew.-%, vorzugsweise 2 Gew.-% bis 4 Gew.-%, bezogen auf die Gesamtzusammensetzung, enthalten sind.

Für entsprechend zusammengesetzte Zusammensetzungen werden erfindungsgemäß besonders gute Wirkeffekte realisiert. Gleichermaßen kann eine günstige bzw. anwendungsorientierte Dosierung umgesetzt werden, was vorteilhaft im Hinblick auf Akzeptanz und Adhärenz sein kann.

**In** einer weiteren bevorzugten Ausführungsform kann die erfindungsgemäße Zusammensetzung ein oder mehrere Adjuvantien enthalten, insbesondere in Mengen in einem Bereich von 0,1 Gew.-% bis 50 Gew.-%, insbesondere 3 Gew.-% bis 30 Gew.-%, vorzugsweise 5 Gew.-% bis 15 Gew.-%, bezogen auf die Gesamtzusammensetzung.

Adjuvantien dienen im Rahmen der Erfindung dazu, die pharmakologische Wirksamkeit, Stabilität, Bioverfügbarkeit oder sensorische Akzeptanz der Zubereitung zu verbessern und können sowohl natürlichen als auch synthetischen Ursprungs sein. Die Adjuvantien werden vorzugsweise so ausgewählt, dass sie mit dem oder den aktiven pharmazeutischen Wirkstoffen chemisch und physikalisch kompatibel sind. Ferner können sie dazu beitragen, die Freisetzungscharakteristik, den Geschmack, die Haltbarkeit oder die Verträglichkeit der Zusammensetzung positiv zu beeinflussen.

**In** einer bevorzugten Ausführungsform umfasst die Zusammensetzung aromatisierende und/oder organoleptische Adjuvantien, die insbesondere der Geschmacksverbesserung, der Maskierung unangenehmer Geschmacksnoten oder der Erzeugung eines kühlenden oder erfrischenden Mundgefühls dienen. Hierzu gehören beispielsweise Menthol, Thymol, Eucalyptol, Pfefferminzöl, Citronensäure, Milchsäure, Laktat, Sucralose, Xylit und Maltit. Diese Adjuvantien können vorteilhaft auch zur Einstellung des pH-Wertes, zur Verbesserung der Auflösung oder zur Stabilisierung hygroskopischer Bestandteile beitragen.

**In** einer weiteren bevorzugten Ausführungsform enthält die Zusammensetzung phytotherapeutische Adjuvantien bzw. pflanzliche Extrakte, die ergänzende pharmakologische Wirkungen entfalten können. Besonders geeignete Phytotherapeutika bzw. pflanzliche Extrakte sind Ingwer-Extrakt, der Gingerole oder Shogaole aufweist und über antiemetische sowie gastroprotektive Eigenschaften verfügt, Ginseng-Extrakt mit adaptogener und tonisierender Wirkung, Guarana-Extrakt und grüner Tee-Extrakt mit stimulierenden und antioxidativen Eigenschaften sowie Aloe-Vera-Extrakt, der eine milde entzündungshemmende und epithelregenerierende Wirkung zeigt. Weitere geeignete pflanzliche Adjuvantien sind Curcumin, Resveratrol, Quercetin, Lutein und/oder Lycopin, welche als polyphenolische Antioxidantien wirken und oxidative Abbauprozesse empfindlicher Wirkstoffe in der Matrix verhindern können. Zusätzlich können ätherische Öle, insbesondere aus Pfefferminze, Fenchel, Kamille, Rosmarin oder Eukalyptus, in der erfindungsgemäßen Zusammensetzung enthalten sein, die durch ihre spasmolytische und aromatische Wirkung sowohl die sensorischen als auch die funktionellen Eigenschaften der Zusammensetzung verbessern.

In einer weiteren bevorzugten Ausführungsform kann die Zusammensetzung Vitamine, Coenzyme und/oder redox-aktive Adjuvantien enthalten, welche die oxidative Stabilität und den physiologischen Nutzen der Formulierung erhöhen. Besonders bevorzugt sind Vitamin B6, Vitamin C (Ascorbinsäure), Vitamin E (Tocopherol), Niacin, Pantothensäure, Folsäure, Vitamin B12 (Cyanocobalamin), Coenzym Q10, Biotin und Riboflavin. Diese Stoffe können einzeln oder in Kombination mit pflanzlichen Polyphenolen eingesetzt werden, um synergistische antioxidative Effekte zu erzielen und den oxidativen Stress in der pharmazeutischen Matrix zu reduzieren.

Ferner kann die Zusammensetzung Aminosäuren, insbesondere auch Peptide oder biogene Derivate dieser, enthalten, welche antioxidative, zellschützende oder stoffwechselmodulierende Eigenschaften aufweisen. Hierzu zählen insbesondere DL-Carnitin, DL-Theanin, DL-Lysin, Taurin, Glutathion und Allantoin. Solche Komponenten können die Bioverfügbarkeit lipophiler oder empfindlicher Wirkstoffe verbessern und unterstützen den physiologischen Energiehaushalt des Anwenders.

Darüber hinaus hat es sich bewährt, wenn die Zusammensetzung ein oder mehrere elektrolytische oder mineralische Adjuvantien enthält. Diese können vorzugsweise ausgewählt sein aus physiologisch verträglichen Salzen von Natrium, Kalium, Calcium, Magnesium und deren Mischungen. Besonders bevorzugt sind hierbei Kaliumchlorid, Natriumchlorid, Natriumcitrat, Calciumcitrat, Magnesiumcitrat und deren Mischungen. Diese Adjuvantien tragen vorteilhaft zu einer ausgewogenen Versorgung bzw. Wiederherstellung der Versorgungsbalance für Mineralstoffe bzw. Elektrolyte bei, welche ihrerseits zu einer Verbesserung der Hangover-Symptomatik beitragen kann. Die genannten Salze unterstützen die Homöostase nach Flüssigkeits- oder Elektrolytverlust, wie sie bei Übermüdung, Dehydratation oder Alkoholaufnahme auftreten können. **In** Form ihrer Citrate können die Salze, insbesondere Magnesium- und Calciumsalze, zusätzlich als pH-regulierende Puffer und Mildalkalisatoren wirken. Magnesium- und Calciumionen unterstützen zudem die neuromuskuläre Stabilität und Kreislaufregulation, während Kaliumionen der Ermüdung des Herz-Kreislauf-Systems entgegenwirken.

Darüber hinaus werden gute Ergebnisse im Hinblick auf eine Milderung der Hangover-Symptomatik realisiert, wenn die Zusammensetzung ein oder mehrere zuckerhaltige oder glukosestabilisierende Adjuvantien enthält. Bevorzugt sind in diesem Zusammenhang Zucker (Saccharose), Dextrose (Glucose) oder andere mono- oder disaccharidische Kohlenhydrate sowie deren Derivate. Diese Zuckerstoffe dienen als schnell verfügbare Energiequelle und unterstützen die physiologische Regeneration nach hypoglykämischen oder alkoholbedingten Zuständen.

**In** einer weiteren Ausführungsform können koffeinhaltige bzw. stimulierende Adjuvantien enthalten sein, insbesondere Koffein, Guarana-Extrakt, Theobromin, Pseudo-Ephedrin und Taurin. Diese Substanzen können zentral stimulierend wirken und in Kombination mit den erfindungsgemäß vorgesehenen Wirkstoffen mit u. a. analgetischer, adaptogener oder anti-fatigue-Wirkung als funktionelle Verstärker dienen.

Die vorgenannten Adjuvantien können einzeln oder in beliebiger Kombination verwendet werden. Durch geeignete Kombination der Adjuvantien kann insbesondere im Zusammenspiel mit den Wirkstoffen der erfindungsgemäßen Zusammensetzung eine synergistische Wirkung erzielt werden, die sowohl die therapeutische Wirksamkeit als auch die Anwenderakzeptanz der erfindungsgemäßen Zusammensetzung verbessert.

Insbesondere die Kombination der Adjuvantien Lysin (DL-Lysin 1 : 1), Pseudo-Ephedrin und/oder Koffein mit den Wirkstoffen der Klassen (i) bis (iii) der erfindungsgemäßen Zusammensetzung, insbesondere mit den Wirkstoffen Ibuprofen, Dimenhydrinat und Cinnarizin, ist im Rahmen der Erfindung besonders bevorzugt, da sich diese als besonders vorteilhaft im Hinblick auf eine Beschleunigung bzw. Verbesserung der intendierten Wirkung im Rahmen der Behandlung von Veisalgia bzw. *Hangovern* erwiesen hat. Insbesondere gestattet diese Kombination eine Verbesserung der Bioverfügbarkeit, einen schnelleren Wirkungseintritt sowie eine physiologische Unterstützung der Kreislauffunktion. Lysin verbessert dabei insbesondere die Löslichkeit und Magenverträglichkeit von sauren Wirkstoffen (bspw. Ibuprofen), während Koffein die analgetische Wirkung verstärken und Müdigkeit mindern kann und Pseudoephedrin eine tonisierende und vasokonstriktorische Komponente beisteuert. Eine entsprechende Zusammensetzung realisiert damit vorteilhaft eine synergistische Analgesie und Leistungsstabilisierung bei gleichzeitig verbesserter Verträglichkeit.

Was weiter die Darreichungs- bzw. Applikationsform der erfindungsgemäßen Zusammensetzung anbelangt, hat es sich bewährt, wenn die Zusammensetzung in Form einer festen Dosierung (feste Dosierungsform), insbesondere für die orale Applikation, vorliegt und/oder wenn die Zusammensetzung eine feste Matrix, insbesondere als pharmazeutischen Träger für die Wirkstoffe, umfasst. Insbesondere kann die in Form einer festen Dosierung (festen Dosierungsform) für die orale Applikation vorliegende Zusammensetzung dabei als Tablette, insbesondere Kautablette, Filmtablette, Dragees und/oder Retardtablette, Kapsel, insbesondere Hartkapsel, und/oder als Granulat und/oder Pulver, insbesondere für die Herstellung wässriger Suspensionen ausgebildet sein. Bevorzugt ist die Ausbildung als Tablette, insbesondere Kautablette, Filmtablette, Dragees und/oder Retardtablette, und/oder Kapsel, insbesondere Hartkapsel. In diesem Zusammenhang hat sich insbesondere auch eine magensaftresistente Ausgestaltung bewährt.

Die erfindungsgemäße Zusammensetzung kann ferner eine insbesondere feste Matrix umfassen, welche die Wirkstoffe enthält bzw. einbettet und in diesem Sinne als Träger-, Freisetzungs- und Stabilisierungssystem dient. Vorzugsweise enthält die Zusammensetzung die Matrix in Mengen in einem Bereich von 2 Gew.-% bis 60 Gew.-%, insbesondere 4 Gew.-% bis 55 Gew.-%, vorzugsweise 5 Gew.-% bis 50 Gew.-%, bezogen auf die Gesamtzusammensetzung.

Die Matrix enthält vorzugsweise einen oder mehrere pharmazeutisch verträgliche Exzipienten (Hilfsstoffe), insbesondere ausgewählt aus der Gruppe von Füllstoffen, Bindemitteln, Zerfallsmitteln, Gleit- und/oder Schmiermitteln, Feuchthalte- und/oder Fließregulierungsmitteln, Filmbildner, Gelbildner und/oder Strukturgeber, lipophile Komponenten, und/oder Mischungen hiervon, bzw. ist vorzugsweise aus diesen gebildet. Hierdurch können die gewünschten technologischen und biopharmazeutischen Eigenschaften der Darreichungsform gesteuert bzw. optimiert werden.

In einer bevorzugten Ausführungsform umfasst die Matrix als Exzipient einen oder mehrere polymere Bestandteile, ausgewählt aus der Gruppe bestehend aus Cellulosederivaten, wie Hydroxypropylmethylcellulose, Hydroxyethylcellulose oder Ethylcellulose, ferner Polyvinylalkohol, Polyvinylpyrrolidon, Polyethylenglykolen, Polylactiden, Polyglycoliden oder deren Copolymeren. Diese Stoffe können - in Abhängigkeit von der gewünschten Freisetzungscharakteristik - als Filmbildner, Gelbildner oder Strukturgeber fungieren und tragen zur mechanischen Festigkeit der festen Einheit bei.

Zur Steuerung der Wirkstofffreisetzung kann die Matrix zusätzlich lipophile Komponenten enthalten, wie gesättigte oder ungesättigte Fettsäuren, Wachse, Glyceride oder hydrierte pflanzliche Öle. Diese erzeugen eine verzögerte Freisetzung durch Diffusions- oder Erosionsmechanismen innerhalb der kompakten Matrixstruktur. Alternativ oder ergänzend können hydrophile Füllstoffe, beispielsweise Lactose, Mannit, Sorbit, Xylit oder mikrokristalline Cellulose, eingesetzt werden, um die Zerfalls- und Wasseraufnahme-Eigenschaften zu modulieren.

Darüber hinaus kann die Matrix als Exzipient ein oder mehrere Bindemittel enthalten, darunter Polyvinylpyrrolidon (Povidon), modifizierte Stärken, Gelatine oder Zuckeralkoholderivate, welche die Kohäsion der Pulverpartikel während der Formgebung erhöhen und die Tablettierbarkeit verbessern.

Zur gezielten Steuerung des Zerfalls und der Desintegration können in der Matrix Zerfallsmittel enthalten sein, insbesondere Croscarmellose-Natrium, Natriumstärkeglykolat, Crospovidon oder mikrokristalline Cellulose. Diese fördern den schnellen Zerfall der Darreichungsform nach oraler Applikation und ermöglichen eine gleichmäßige Wirkstofffreisetzung.

Für eine Verbesserung der Fließfähigkeit, zur Reduktion von Reibung während der Tablettierung und im Sinne einer Optimierung der Oberflächenbeschaffenheit können ein oder mehrere Gleit- und/oder Schmiermittel eingesetzt werden.

Bevorzugte Vertreter sind Magnesiumstearat, Stearinsäure, Talkum und hochdisperse Kieselsäure.

Die Matrix kann ferner Feuchthalte- und/oder Fließregulierungsmittel enthalten, insbesondere Polyethylenglykole, Glycerol, Sorbitol oder Mannit. Diese tragen zur Stabilisierung hygroskopischer Wirkstoffe bei und beeinflussen die Plastizität und Kompressionsfähigkeit der Tablettenmischung positiv.

Für retardierte oder kontrollierte Freisetzungssysteme kann die Matrix zusätzlich quellfähige oder porenbildende Bestandteile enthalten. Geeignet sind insbesondere Natriumcarboxymethylcellulose, vernetzte Polyacrylsäuren, Alginate, Carrageenane oder Polysaccharidkomplexe, die bei Kontakt mit wässrigen Medien ein Gel oder Netzwerk bilden und so eine graduelle Freisetzung des Wirkstoffs ermöglichen.

Zur Stabilisierung empfindlicher Wirkstoffe kann die Matrix ferner pH-Puffer, Antioxidantien, Feuchtigkeitsregulatoren oder Komplexbildner enthalten. Bevorzugte Vertreter sind Ascorbinsäure, Butylhydroxytoluol, Silicagel, Mannit oder Cyclodextrine. Diese Zusätze tragen zur Erhaltung der chemischen und physikalischen Integrität der Wirkstoffe während der Lagerung und Anwendung bei.

**In** einer bevorzugten Ausführungsform ist die Matrix homogen dispergiert, wobei der Wirkstoff molekular gelöst oder fein verteilt vorliegt. Alternativ kann der Wirkstoff in einer mehrphasigen Struktur eingelagert sein, bestehend aus einer diffusionshemmenden Außenschicht und einem erodierbaren oder löslichen Kern. Durch geeignete Wahl und Kombination der Matrixbestandteile lassen sich Freisetzungsprofile von sofortiger bis zu verzögerter (retardierter) Wirkstofffreisetzung einstellen.

Die Matrix kann ferner funktionale Beschichtungen oder Überzüge enthalten, die als Feuchtigkeitsbarriere, Geschmacksmaske oder als pH-abhängige Schutzschicht dienen. Geeignete Polymere sind insbesondere Eudragit^{®}-Typen, Celluloseacetatphthalat oder Poly(meth)acrylate, die in Lösung oder Dispersion aufgebracht werden.

Insgesamt ermöglicht die Auswahl und Kombination der beschriebenen Exzipienten und Matrixbestandteile eine gezielte Steuerung der mechanischen Festigkeit, Disintegration, Wirkstofffreisetzung und Stabilität der festen pharmazeutischen Zubereitung, wobei die Zusammensetzung vorteilhaft so ausgelegt ist, dass sämtliche Bestandteile pharmakologisch unbedenklich und mit den Wirkstoffen kompatibel sind.

Darüber hinaus hat es sich als zweckmäßig erwiesen, wenn die Zusammensetzung, insbesondere als feste Dosierungsform, pro Dosis ein Gesamtgewicht im Bereich von 0,25 g bis 5 g, insbesondere im Bereich von 0,4 g bis 4 g, vorzugsweise im Bereich von 0,5 g bis 3,5 g, aufweist.

Für ein optimierte Relation zwischen Einzeldosis und Wirkeffektivität hat es sich bewährt, wenn die Zusammensetzung, insbesondere als feste Dosierungsform, pro Dosis Klasse (i)-Wirkstoffe in Mengen in einem Bereich von 25 mg bis 900 mg, insbesondere 100 mg bis 800 mg, vorzugsweise 150 mg bis 650 mg, enthält, und/oder wenn die Zusammensetzung pro Dosis Klasse (ii)-Wirkstoffe in Mengen in einem Bereich von 10 mg bis 100 mg, insbesondere 20 mg bis 70 mg, vorzugsweise 30 mg bis 50 mg, enthält, und/oder wenn die Zusammensetzung pro Dosis Klasse (iii)-Wirkstoffe in Mengen in einem Bereich von 5 mg bis 50 mg, insbesondere 10 mg bis 30 mg, vorzugsweise 15 mg bis 25 mg, enthält. In den vorgerannten Mengenbereichen für die jeweiligen Wirkstoffe werden die eingangs beschriebenen vorteilhaften Wirkeffekte vorteilhaft in besonderem Maße realisiert.

Darüber hinaus werden gute Ergebnisse bezüglich der Linderung der *Hangover-*Symptomatik erhalten, wenn die Zusammensetzung, bezogen auf die Wirkstoffe insgesamt, mit einer Tagesdosis im Bereich von 50 mg/diem bis 2.000 mg/diem, insbesondere 250 mg/diem bis 1.600 mg/diem, vorzugsweise 400 mg/diem bis 1.400 mg/diem, verabreicht wird, und/oder wenn die Zusammensetzung als feste Dosierungsform, bezogen auf die Wirkstoffe insgesamt, zur Verabreichung einer Tagesdosis im Bereich von 50 mg/diem bis 2.000 mg/diem, insbesondere 250 mg/diem bis 1.600 mg/diem, vorzugsweise 400 mg/diem bis 1.400 mg/diem, hergerichtet ist.

Gemäß einer bevorzugten Ausgestaltung der vorliegenden Erfindung umfasst die vorliegende Erfindung gemäß dem geltenden Aspekt ferner insbesondere auch eine solche Zusammensetzung zur Verwendung bei der prophylaktischen und/oder therapeutischen Behandlung von Veisalgia,
welche jeweils eine wirksame, insbesondere pharmazeutisch und/oder therapeutisch wirksame, Menge von Wirkstoffen aus den Wirkstoffklassen (i) nichtsteroidales Antirheumatikum, (ii) H₁-Antihistaminikum und (iii) Calciumkanal-Antagonist umfasst, wobei die Wirkstoffe ausgewählt sind aus
Klasse (i): Acetylsalicylsäure, Ibuprofen, Dexibuprofen, Flurbiprofen, Naproxen, Ketoprofen, Tiaprofensäure, Diclofenac, Indometacin, Acemetacin, Flufenaminsäure, Mefenaminsäure, Piroxicam, Tenoxicam, Meloxicam, Lornoxicam, Nabumeton und/oder Mischungen hiervon,
Klasse (ii): Dimenhydrinat, und
Klasse (iii): Cinnarizin,
   und
welche ferner Adjuvantien ausgewählt aus der Gruppe von aromatisierenden und/oder organoleptischen Adjuvantien, phytotherapeutischen Adjuvantien, Pflanzenextrakten, Vitaminen, Coenzymen, redox-aktiven Adjuvantien, Aminosäuren, koffeinhaltigen Adjuvantien, stimulierenden Adjuvantien und/oder Mischungen hiervor umfasst, und/oder
welche in Form einer festen Dosierung (feste Dosierungsform), insbesondere für die orale Applikation, vorliegt und/oder wobei die Zusammensetzung eine feste Matrix, insbesondere als pharmazeutischen Träger für die Wirkstoffe, umfasst.

Die zuvor genannten jeweiligen Vorteile und Besonderheiten gelten für diese bevorzugte Ausführung der vorliegenden Erfindung gleichermaßen.

Für weitergehende Einzelheiten zu diesem Erfindungsaspekt kann Bezug genommen werden auf die Ausführungen zu den weiteren erfindungsgemäßen Aspekten, wobei diese Ausführungen gleichermaßen für den vorliegenden Erfindungsaspekt gelten.

Weiterer Gegenstand der vorliegenden Erfindung - gemäß einem **zweiten** und **dritten** Aspekt der vorliegenden Erfindung - ist zudem die Verwendung einer Zusammensetzung, insbesondere pharmazeutischen Zusammensetzung, nach der vorliegenden Erfindung zur Herstellung eines Arzneimittels oder Medikaments zur prophylaktischen und/oder therapeutischen Behandlung von Veisalgia., sowie ein Arzneimittel oder Medikament zur prophylaktischen und/oder therapeutischen Behandlung von Veisalgia umfassend eine Zusammensetzung nach der vorliegenden Erfindung.

Für weitergehende Einzelheiten zu diesem Erfindungsaspekt kann Bezug genommen werden auf die Ausführungen zu den weiteren erfindungsgemäßen Aspekten, wobei diese Ausführungen gleichermaßen für den vorliegenden Erfindungsaspekt gelten.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, jedoch ohne die vorliegende Erfindung hierauf beschränken zu wollen.

### AUSFÜHRUNGSBEISPIELE:

Zur Evaluierung der Wirkung der erfindungsgemäßen Zusammensetzung wurde eine kontrollierte, einfach verblindete Vergleichsstudie mit freiwilligen Probanden durchgeführt.

### Versuchsaufbau:

Zwei Gruppen (Gruppe A (Verum-Gruppe) und Gruppe B (Vergleichsgruppe)) mit jeweils 20 Probanden im Alter zwischen 25 und 55 Jahren wurden gebildet.

Vor Versuchsbeginn wurden alle Probanden zu ihrem allgemeinen Befinden befragt und berichteten sodann über Befindlichkeitsstörungen wie Kopfschmerzen, Müdigkeit, allgemeine Abgeschlagenheit, Übelkeit oder gastrointestinale Beschwerden leichten bis mittleren Grades.

Alle Probanden konsumierten nach Dokumentation des Befindens über den Zeitraum eines Abends (ca. 3 bis 5 Stunden) und zeitlich vorgelagert zur Einnahme der erfindungsgemäßen Zusammensetzung unter Aufsicht eine vorgegebene, am individuellen Körpergewicht ausgerichtete Menge Alkoholika.

Nach erfolgter Alkoholaufnahme wurde den Probanden folgende Dosierung für die orale Applikation in Tablettenform verabreicht:
*1. Gruppe A (Verum-Gruppe):*
   Die Probanden der Gruppe A erhielten eine Einzeldosis der erfindungsgemäßen Zusammensetzung, enthaltend
      400 mg Ibuprofen-Lysin (DL 1:1),
      20 mg Cinnarizin,
      40 mg Dimenhydrinat,
      200 mg Matrixkomponenten gemäß der Erfindung.
   Die Einnahme der Zusammensetzung erfolgte am späten Abend, unmittelbar vor dem Zubettgehen, etwa 30 bis 60 Minuten nach der letzten Alkoholaufnahme, mit 200 ml Wasser.
   Nach der Einnahme begaben sich die Probanden in eine Ruhe- bzw. Schlafphase. Die subjektive Befindlichkeit wurde durch die Probanden nach dem Erwachen am nächsten Morgen anhand einer qualitativen Positiv-/Negativ-Skala dokumentiert.
*2. Gruppe B (Vergleichsgruppe):*
   Die Probanden der Gruppe B erhielten kein Therapeutikum und lediglich auf Rückfrage im Wege einer Selbstmedikation die Möglichkeit, auf herkömmliche Scherzmittel wie Paracetamol, Ibuprofen oder Metamizol (Novalgin^{®}) zurückzugreifen.
   Sofern eine Einnahme erfolgte, fand diese am späten Abend, unmittelbar vor dem Zubettgehen, etwa 30 bis 60 Minuten nach der letzten Alkoholaufnahme, mit 200 ml Wasser statt. Etwa 20 % der Probanden ergriffen diese Möglichkeit der Selbstmedikation.
   Die Probanden begaben sich etwa 60 bis 90 Minuten nach der letzten Alkoholaufnahme in eine Ruhe- bzw. Schlafphase. Die subjektive Befindlichkeit wurde durch die Probanden nach dem Erwachen am nächsten Morgen anhand einer qualitativen Positiv-/Negativ-Skala dokumentiert.
*3. Bewertungsmethode*
   Die Probanden wurden gebeten, ihr Befinden nach dem Erwachen am nächsten Morgen in Bezug auf fünf Hauptsymptome anzugeben:
      ▪ Kopfschmerz / Druckgefühl im Kopf
      ▪ Übelkeit / Magenunruhe
      ▪ Allgemeines Wohlbefinden / Wachheit
      ▪ Kreislaufstabilität / Schwindelgefühl
      ▪ Stimmung / Leistungsbereitschaft
   Die Auswertung erfolgte durch Selbstbewertung mit den Symbolen:
      "++" = deutlich bessere Symptomatik als üblich,
      "+" = bessere Symptomatik als üblich,
      "-" = schlechtere Symptomatik als üblich,
      "0" = unveränderte Symptomatik.
   Die Bewertungen wurden dokumentiert und anschließend anonymisiert ausgewertet.
*4. Ergebnisse*
   Nach dem Erwachen am nächsten Morgen gaben in der Verum-Gruppe A über 80 % der Probanden mindestens zwei positive Veränderungen ("+" oder "++") gegenüber der individuell üblicherweise zu erwartenden *Hangover-*Symptomatik an, während in der Vergleichsgruppe B kaum über eine vergleichbare Besserung berichtet wurde.
   Insbesondere stellte sich in Gruppe A eine kombinierte Besserung sowohl von Symptomen wie Kopfschmerz bzw. Druckgefühl im Kopf und Übelkeit bzw. Magenunruhe sowie Kreislaufstabilität bzw. Schwindelgefühl, d.h. ein insgesamt deutlich geringeres Gefühl der Abgeschlagenheit als üblich, ein, während in Gruppe B überwiegend nur eine Besserung im Wesentlichen eines der Symptome festgestellt wurde (Kopfschmerzen bzw. Druckgefühl im Kopf, insbesondere im Wesentlichen im Falle der Probanden, die ein herkömmliches Schmerzmittel eingenommen hatten).
   Bei weiterer Beobachtung über den Tagesverlauf zeigten in der Verum-Gruppe über 85 % der Probanden ein positives oder deutlich positives Befinden ("+" bzw. "++"), während in der Vergleichsgruppe nur eine moderate und im Wesentlichen der individuell gewohnten Hangover-Symptomatik folgende Verbesserung angegeben wurde. In Gruppe A konnte im Tagesverlauf zudem eine stabile oder eine weiter zunehmende bzw. sich weiter ausbreitende Verbesserung der Symptomatik insbesondere auch für die Kriterien Allgemeines Wohlbefinden bzw. Wachheit und Stimmung bzw. Leistungsbereitschaft festgestellt werden.
*5. Weitere Untersuchungen*
   Die Vergleichsstudie wurde für die erfindungsgemäße Zusammensetzung wiederholt, wobei die Probanden der Verum-Gruppe A die Zusammensetzung im Rahmen der weiteren Vergleichsstudie erst ca. 30 Minuten nach dem Erwachen am nächsten Morgen einnahmen. Es wurde weiterhin eine spürbare Verbesserung der Hangover-Symptomatik beobachtet, die insbesondere auch ausgeprägter war als die Verbesserung der Symptomatik mit herkömmlichen Schmerzmitteln allein. Im Vergleich der Applikationsschemata wurde eine verbesserte Wirkung der erfindungsgemäßen Zusammensetzung festgestellt, wenn diese 30 bis 60 Minuten nach der letzten Alkoholaufnahme eingenommen wird. Es wird in diese Fall eine umfassendere Linderung der Hangover-Symptomatik beobachtet. Insbesondere bei einer ausgeprägten Hangover-Symptomatik bietet sich gleichwohl vorteilhaft ein kombiniertes Applikationsschema aus Einnahme der erfindungsgemäßen Zusammensetzung vor dem Zubettgehen sowie nach dem Erwachen am nächsten Morgen an.
   Die Vergleichsstudie wurde zudem auch für eine erfindungsgemäße Zusammensetzung, die zusätzlich auch Koffein als Adjuvant enthält, wiederholt. Es wurden vergleichbare Ergebnisse erzielt, wobei insbesondere eine noch weitergehende Verbesserung in Bezug auf die die Kriterien Allgemeines Wohlbefinden bzw. Wachheit und Stimmung bzw. Leistungsbereitschaft angegeben wurden.

## Patentansprüche

1. Zusammensetzung, insbesondere pharmazeutische Zusammensetzung, zur Verwendung bei der prophylaktischen und/oder therapeutischen (symptomatischen) Behandlung von Veisalgia (Alkoholintoxikation, *Hangover),*
wobei die Zusammensetzung jeweils eine wirksame, insbesondere pharmazeutisch und/oder therapeutisch wirksame, Menge von Wirkstoffen aus den Wirkstoffklassen (i) nichtsteroidales Antirheumatikum (NSAR, *non-steroidal anti-inflammatory drug,* NSAID), (ii) H₁-Antihistaminikum und (iii) Calciumkanal-Antagonist umfasst, wobei die Wirkstoffe ausgewählt sind aus
Klasse (i): Acetylsalicylsäure, Ibuprofen, Dexibuprofen, Flurbiprofen, Naproxen, Ketoprofen, Tiaprofensäure, Diclofenac, Indometacin, Acemetacin, Flufenaminsäure, Mefenaminsäure, Piroxicam, Tenoxicam, Meloxicam, Lornoxicam, Nabumeton und/oder Mischungen hiervon,
Klasse (ii): Dimenhydrinat, und
Klasse (iii): Cinnarizin.

2. Zusammensetzung zur Verwendung nach Anspruch 1, wobei die Zusammensetzung die Wirkstoffe der Klassen (i) und (ii) in gewichtsbezogen Mengenverhältnissen Klasse (i) : Klasse (ii) im Bereich von 1 : 2 bis 20 : 1, insbesondere 2 : 1 bis 15 : 1, vorzugsweise 5 : 1 bis 10 : 1, enthält, und/oder
wobei die Zusammensetzung die Wirkstoffe der Klassen (i) und (iii) in gewichtsbezogen Mengenverhältnissen Klasse (i) : Klasse (iii) im Bereich von 1 : 1 bis 30 : 1, insbesondere 3 : 1 bis 25 : 1, vorzugsweise 10 : 1 bis 20 : 1, enthält, und/oder
wobei die Zusammensetzung die Wirkstoffe der Klassen (ii) und (iii) in gewichtsbezogen Mengenverhältnissen Klasse (ii) : Klasse (iii) im Bereich von 5 : 1 bis 1 : 1, insbesondere 3 : 1 bis 1 : 1,25, vorzugsweise 2,5 : 1 bis 1,5 : 1, enthält.

3. Zusammensetzung zur Verwendung nach Anspruch 1 oder 2, wobei die Zusammensetzung die Wirkstoffe der Klassen (i), (ii) und (iii) in gewichtsbezogen Mengenverhältnissen Klasse (i) : Klasse (ii) : Klasse (iii) im Bereich von 30 : 3 : 1 bis 3 : 1 : 1, insbesondere 25 : 2,5 : 1 bis 10 : 1,25 : 1, vorzugsweise 20 : 2,5 : 1 bis 15 : 1,5 : 1, enthält.

4. Zusammensetzung zur Verwendung nach einem der vorangehenden Ansprüche, wobei der Wirkstoff der Klasse (i) ausgewählt ist aus Acetylsalicylsäure, Ibuprofen, Naproxen, Diclofenac und/oder Mischungen hiervon, insbesondere Ibuprofen ist.

5. Zusammensetzung zur Verwendung nach einem der vorangehenden Ansprüche, wobei die Zusammensetzung die Wirkstoffe insgesamt in Mengen in einem Bereich von 40 Gew.-% bis 98 Gew.-%, insbesondere 45 Gew.-% bis 96 Gew.-%, vorzugsweise 50 Gew.-% bis 95 Gew.-%, bezogen auf die Gesamtzusammensetzung, enthält.

6. Zusammensetzung zur Verwendung nach einem der vorangehenden Ansprüche, wobei die Zusammensetzung Wirkstoffe der Klasse (i) in Mengen in einem Bereich von 30 Gew.-% bis 75 Gew.-%, insbesondere 40 Gew.-% bis 70 Gew.-%, vorzugsweise 50 Gew.-% bis 65 Gew.-%, bezogen auf die Gesamtzusammensetzung, enthält, und/oder
wobei die Zusammensetzung Wirkstoffe der Klasse (ii) in Mengen in einem Bereich von 4 Gew.-% bis 20 Gew.-%, insbesondere 5 Gew.-% bis 15 Gew.-%, vorzugsweise 6 Gew.-% bis 10 Gew.-%, bezogen auf die Gesamtzusammensetzung, enthält, und/oder
wobei die Zusammensetzung Wirkstoffe der Klasse (iii) in Mengen in einem Bereich von 0,5 Gew.-% bis 7 Gew.-%, insbesondere 1,5 Gew.-% bis 5 Gew.-%, vorzugsweise 2 Gew.-% bis 4 Gew.-%, bezogen auf die Gesamtzusammensetzung, enthält.

7. Zusammensetzung zur Verwendung nach einem der vorangehenden Ansprüche, wobei die Zusammensetzung ein oder mehrere Adjuvantien enthält,
insbesondere wobei die Zusammensetzung Adjuvantien in Mengen in einem Bereich von 0,1 Gew.-% bis 50 Gew.-%, insbesondere 3 Gew.-% bis 30 Gew.-%, vorzugsweise 5 Gew.-% bis 15 Gew.-%, bezogen auf die Gesamtzusammensetzung, enthält.

8. Zusammensetzung zur Verwendung nach einem der vorangehenden Ansprüche, wobei die Adjuvantien ausgewählt sind aus der Gruppe von aromatisierenden und/oder organoleptischen Adjuvantien, phytotherapeutischen Adjuvantien, Pflanzenextrakten, Vitaminen, Coenzymen, redox-aktiven Adjuvantien, Aminosäuren, koffeinhaltigen Adjuvantien, stimulierenden Adjuvantien und/oder Mischungen hiervor.

9. Zusammensetzung zur Verwendung nach einem der vorangehenden Ansprüche, wobei die Zusammensetzung in Form einer festen Dosierung (feste Dosierungsform), insbesondere für die orale Applikation, vorliegt und/oder wobei die Zusammensetzung eine feste Matrix, insbesondere als pharmazeutischen Träger für die Wirkstoffe, umfasst.

10. Zusammensetzung zur Verwendung nach einem der vorangehenden Ansprüche, wobei die die Zusammensetzung die Matrix in Mengen in einem Bereich von 2 Gew.-% bis 60 Gew.-%, insbesondere 4 Gew.-% bis 55 Gew.-%, vorzugsweise 5 Gew.-% bis 50 Gew.-%, bezogen auf die Gesamtzusammensetzung, enthält, und/oder
wobei die Matrix einen oder mehrere pharmazeutisch verträgliche Exzipienten, insbesondere ausgewählt aus der Gruppe von Füllstoffen, Bindemitteln, Zerfallsmitteln, Gleit- und/oder Schmiermitteln, Feuchthalte- und/oder Fließregulierungsmitteln, Filmbildner, Gelbildner und/oder Strukturgeber, lipophile Komponenten, und/oder Mischungen hiervon, enthält, insbesondere hieraus gebildet ist.

11. Zusammensetzung zur Verwendung nach einem der vorangehenden Ansprüche, wobei die Zusammensetzung als feste Dosierungsform als Tablette, insbesondere Filmtablette, nicht überzogene Tablette oder magensaftresistente Tablette, ausgebildet ist, und/oder
wobei die Zusammensetzung, insbesondere als feste Dosierungsform, pro Dosis ein Gesamtgewicht im Bereich von 0,25 g bis 5 g, insbesondere im Bereich von 0,4 g bis 4 g, vorzugsweise im Bereich von 0,5 g bis 3,5 g, aufweist.

12. Zusammensetzung zur Verwendung nach einem der vorangehenden Ansprüche, wobei die Zusammensetzung, insbesondere als feste Dosierungsform, pro Dosis Klasse (i)-Wirkstoffe in Mengen in einem Bereich von 25 mg bis 900 mg, insbesondere 100 mg bis 800 mg, vorzugsweise 150 mg bis 650 mg, enthält, und/oder
wobei die Zusammensetzung pro Dosis Klasse (ii)-Wirkstoffe in Mengen in einem Bereich von 10 mg bis 100 mg, insbesondere 20 mg bis 70 mg, vorzugsweise 30 mg bis 50 mg, enthält, und/oder
wobei die Zusammensetzung pro Dosis Klasse (iii)-Wirkstoffe in Mengen in einem Bereich von 5 mg bis 50 mg, insbesondere 10 mg bis 30 mg, vorzugsweise 15 mg bis 25 mg, enthält.

13. Zusammensetzung zur Verwendung nach einem der vorangehenden Ansprüche, wobei die Zusammensetzung, bezogen auf die Wirkstoffe insgesamt, mit einer Tagesdosis im Bereich von 50 mg/diem bis 2.000 mg/diem, insbesondere 250 mg/diem bis 1.600 mg/diem, vorzugsweise 400 mg/diem bis 1.400 mg/diem, verabreicht wird, und/oder
wobei die Zusammensetzung als feste Dosierungsform, bezogen auf die Wirkstoffe insgesamt, zur Verabreichung einer Tagesdosis im Bereich von 50 mg/diem bis 2.000 mg/diem, insbesondere 250 mg/diem bis 1.600 mg/diem, vorzugsweise 400 mg/diem bis 1.400 mg/diem, hergerichtet ist.

14. Verwendung einer Zusammensetzung, insbesondere pharmazeutischen Zusammensetzung, vorzugsweise nach einem der vorangehenden Ansprüche, zur Herstellung eines Arzneimittels oder Medikaments zur prophylaktischen und/oder therapeutischen Behandlung von Veisalgia.

15. Arzneimittel oder Medikament zur prophylaktischen und/oder therapeutischen Behandlung von Veisalgia umfassend eine Zusammensetzung nach einem oder mehreren der diesbezüglichen vorangehenden Ansprüche.

## Geänderte Patentansprüche

### Geänderte Patentansprüche gemäss Regel 137(2) EPÜ.

1. Zusammensetzung, insbesondere pharmazeutische Zusammensetzung, zur Verwendung bei der prophylaktischen und/oder therapeutischen (symptomatischen) Behandlung von Veisalgia (Alkoholintoxikation, *Hangover*),
wobei die Zusammensetzung jeweils eine wirksame, insbesondere pharmazeutisch und/oder therapeutisch wirksame, Menge von Wirkstoffen aus den Wirkstoffklassen (i) nichtsteroidales Antirheumatikum (NSAR, *non-steroidal anti-inflammatory drug,* NSAID), (ii) H₁-Antihistaminikum und (iii) Calciumkanal-Antagonist umfasst, wobei die Wirkstoffe ausgewählt sind aus
| | |
|---|---|
| Klasse (i): | Acetylsalicylsäure, Ibuprofen, Dexibuprofen, Flurbiprofen, Naproxen, Ketoprofen, Tiaprofensäure, Diclofenac, Indometacin, Acemetacin, Flufenaminsäure, Mefenaminsäure, Piroxicam, Tenoxicam, Meloxicam, Lornoxicam, Nabumeton und/oder Mischungen hiervon, |
| Klasse (ii): | Dimenhydrinat, und |
| Klasse (iii): | Cinnarizin, |
wobei die Zusammensetzung die Wirkstoffe der Klassen (i) und (iii) in gewichtsbezogen Mengenverhältnissen Klasse (i) : Klasse (iii) im Bereich von 1 : 1 bis 30 : 1 enthält, und
wobei die Zusammensetzung die Wirkstoffe der Klassen (ii) und (iii) in gewichtsbezogen Mengenverhältnissen Klasse (ii) : Klasse (iii) im Bereich von 5 : 1 bis 1 : 1 enthält.

2. Zusammensetzung zur Verwendung nach Anspruch 1, wobei die Zusammensetzung die Wirkstoffe der Klassen (i) und (ii) in gewichtsbezogen Mengenverhältnissen Klasse (i) : Klasse (ii) im Bereich von 1 : 2 bis 20 : 1, insbesondere 2 : 1 bis 15 : 1, vorzugsweise 5 : 1 bis 10 : 1, enthält, und/oder
wobei die Zusammensetzung die Wirkstoffe der Klassen (i) und (iii) in gewichtsbezogen Mengenverhältnissen Klasse (i) : Klasse (iii) im Bereich von 3 : 1 bis 25 : 1, vorzugsweise 10 : 1 bis 20 : 1, enthält, und
wobei die Zusammensetzung die Wirkstoffe der Klassen (ii) und (iii) in gewichtsbezogen Mengenverhältnissen Klasse (ii) : Klasse (iii) im Bereich von 3 : 1 bis 1 : 1,25, vorzugsweise 2,5 : 1 bis 1,5 : 1, enthält.

3. Zusammensetzung zur Verwendung nach Anspruch 1 oder 2, wobei die Zusammensetzung die Wirkstoffe der Klassen (i), (ii) und (iii) in gewichtsbezogen Mengenverhältnissen Klasse (i) : Klasse (ii) : Klasse (iii) im Bereich von 30 : 3 : 1 bis 3 : 1 : 1, insbesondere 25 : 2,5 : 1 bis 10 : 1,25 : 1, vorzugsweise 20 : 2,5 : 1 bis 15 : 1,5 : 1, enthält.

4. Zusammensetzung zur Verwendung nach einem der vorangehenden Ansprüche, wobei der Wirkstoff der Klasse (i) ausgewählt ist aus Acetylsalicylsäure, Ibuprofen, Naproxen, Diclofenac und/oder Mischungen hiervon, insbesondere Ibuprofen ist.

5. Zusammensetzung zur Verwendung nach einem der vorangehenden Ansprüche, wobei die Zusammensetzung die Wirkstoffe insgesamt in Mengen in einem Bereich von 40 Gew.-% bis 98 Gew.-%, insbesondere 45 Gew.-% bis 96 Gew.-%, vorzugsweise 50 Gew.-% bis 95 Gew.-%, bezogen auf die Gesamtzusammensetzung, enthält.

6. Zusammensetzung zur Verwendung nach einem der vorangehenden Ansprüche, wobei die Zusammensetzung Wirkstoffe der Klasse (i) in Mengen in einem Bereich von 30 Gew.-% bis 75 Gew.-%, insbesondere 40 Gew.-% bis 70 Gew.-%, vorzugsweise 50 Gew.-% bis 65 Gew.-%, bezogen auf die Gesamtzusammensetzung, enthält, und/oder
wobei die Zusammensetzung Wirkstoffe der Klasse (ii) in Mengen in einem Bereich von 4 Gew.-% bis 20 Gew.-%, insbesondere 5 Gew.-% bis 15 Gew.-%, vorzugsweise 6 Gew.- % bis 10 Gew.-%, bezogen auf die Gesamtzusammensetzung, enthält, und/oder
wobei die Zusammensetzung Wirkstoffe der Klasse (iii) in Mengen in einem Bereich von 0,5 Gew.-% bis 7 Gew.-%, insbesondere 1,5 Gew.-% bis 5 Gew.-%, vorzugsweise 2 Gew.- % bis 4 Gew.-%, bezogen auf die Gesamtzusammensetzung, enthält.

7. Zusammensetzung zur Verwendung nach einem der vorangehenden Ansprüche, wobei die Zusammensetzung ein oder mehrere Adjuvantien enthält,
insbesondere wobei die Zusammensetzung Adjuvantien in Mengen in einem Bereich von 0,1 Gew.-% bis 50 Gew.-%, insbesondere 3 Gew.-% bis 30 Gew.-%, vorzugsweise 5 Gew.-% bis 15 Gew.-%, bezogen auf die Gesamtzusammensetzung, enthält.

8. Zusammensetzung zur Verwendung nach einem der vorangehenden Ansprüche, wobei die Adjuvantien ausgewählt sind aus der Gruppe von aromatisierenden und/oder organoleptischen Adjuvantien, phytotherapeutischen Adjuvantien, Pflanzenextrakten, Vitaminen, Coenzymen, redox-aktiven Adjuvantien, Aminosäuren, koffeinhaltigen Adjuvantien, stimulierenden Adjuvantien und/oder Mischungen hiervor.

9. Zusammensetzung zur Verwendung nach einem der vorangehenden Ansprüche, wobei die Zusammensetzung in Form einer festen Dosierung (feste Dosierungsform), insbesondere für die orale Applikation, vorliegt und/oder wobei die Zusammensetzung eine feste Matrix, insbesondere als pharmazeutischen Träger für die Wirkstoffe, umfasst.

10. Zusammensetzung zur Verwendung nach einem der vorangehenden Ansprüche, wobei die die Zusammensetzung die Matrix in Mengen in einem Bereich von 2 Gew.-% bis 60 Gew.-%, insbesondere 4 Gew.-% bis 55 Gew.-%, vorzugsweise 5 Gew.-% bis 50 Gew.-%, bezogen auf die Gesamtzusammensetzung, enthält, und/oder
wobei die Matrix einen oder mehrere pharmazeutisch verträgliche Exzipienten, insbesondere ausgewählt aus der Gruppe von Füllstoffen, Bindemitteln, Zerfallsmitteln, Gleit- und/oder Schmiermitteln, Feuchthalte- und/oder Fließregulierungsmitteln, Filmbildner, Gelbildner und/oder Strukturgeber, lipophile Komponenten, und/oder Mischungen hiervon, enthält, insbesondere hieraus gebildet ist.

11. Zusammensetzung zur Verwendung nach einem der vorangehenden Ansprüche, wobei die Zusammensetzung als feste Dosierungsform als Tablette, insbesondere Filmtablette, nicht überzogene Tablette oder magensaftresistente Tablette, ausgebildet ist, und/oder
wobei die Zusammensetzung, insbesondere als feste Dosierungsform, pro Dosis ein Gesamtgewicht im Bereich von 0,25 g bis 5 g, insbesondere im Bereich von 0,4 g bis 4 g, vorzugsweise im Bereich von 0,5 g bis 3,5 g, aufweist.

12. Zusammensetzung zur Verwendung nach einem der vorangehenden Ansprüche, wobei die Zusammensetzung, insbesondere als feste Dosierungsform, pro Dosis Klasse (i)-Wirkstoffe in Mengen in einem Bereich von 25 mg bis 900 mg, insbesondere 100 mg bis 800 mg, vorzugsweise 150 mg bis 650 mg, enthält, und/oder
wobei die Zusammensetzung pro Dosis Klasse (ii)-Wirkstoffe in Mengen in einem Bereich von 10 mg bis 100 mg, insbesondere 20 mg bis 70 mg, vorzugsweise 30 mg bis 50 mg, enthält, und/oder
wobei die Zusammensetzung pro Dosis Klasse (iii)-Wirkstoffe in Mengen in einem Bereich von 5 mg bis 50 mg, insbesondere 10 mg bis 30 mg, vorzugsweise 15 mg bis 25 mg, enthält.

13. Zusammensetzung zur Verwendung nach einem der vorangehenden Ansprüche, wobei die Zusammensetzung, bezogen auf die Wirkstoffe insgesamt, mit einer Tagesdosis im Bereich von 50 mg/diem bis 2.000 mg/diem, insbesondere 250 mg/diem bis 1.600 mg/diem, vorzugsweise 400 mg/diem bis 1.400 mg/diem, verabreicht wird, und/oder
wobei die Zusammensetzung als feste Dosierungsform, bezogen auf die Wirkstoffe insgesamt, zur Verabreichung einer Tagesdosis im Bereich von 50 mg/diem bis 2.000 mg/diem, insbesondere 250 mg/diem bis 1.600 mg/diem, vorzugsweise 400 mg/diem bis 1.400 mg/diem, hergerichtet ist.

14. Arzneimittel oder Medikament in der Verwendung zur prophylaktischen und/oder therapeutischen Behandlung von Veisalgia umfassend eine Zusammensetzung nach einem oder mehreren der diesbezüglichen vorangehenden Ansprüche.
